# FASCICULE DE BREVET EUROPEEN

(11) **EP 2 260 002 B1**
(45) Date de publication et mention de la délivrance du brevet: **03.08.2011**
(21) Numéro de dépôt: 09730979.3
(22) Date de dépôt: 20.03.2009
(51) Int. Cl.: C01C 3/02, C01C 3/10, C07C 319/20, C07C 253/00, C07C 253/10

(54) **PROCEDE DE PRODUCTION D'ACIDE CYANHYDRIQUE**
VERFAHREN ZUR HERSTELLUNG VON HYDROZYANSÄURE
PROCESS FOR PRODUCING HYDROCYANIC ACID

(30) Priorité: 20.03.2008 FR 0851807; 01.07.2008 FR 0854437
(43) Date de publication de la demande: 15.12.2010
(73) Titulaire: ARKEMA FRANCE, 92700 Colombes (FR)
(72) Inventeur: FORMENTIN, Enzo, I-30033 Noale (IT); SCHAFER, Catherine, F-57880 Guerting (FR)
(86) Numéro de dépôt international: PCT/FR2009/050472
(87) Numéro de publication internationale: WO 2009/125101

(56) Documents cités:
- EP-A- 1 371 632
- FR-A- 1 261 058
- FR-A- 1 503 622
- FR-A- 2 829 763
- FR-A- 2 903 690
- GB-A- 941 486
- US-A- 3 102 001
- US-A- 3 102 269

## Description

La présente invention concerne la fabrication de l'acide cyanhydrique et a plus particulièrement pour objet un procédé amélioré de production d'acide cyanhydrique par réaction de l'ammoniac sur du méthane dans lequel on met en oeuvre un composé soufré appartenant à la famille des polysulfures, tel que le diméthyldisulfure.

L'acide cyanhydrique HCN trouve de nombreuses applications comme réactif dans divers procédés de synthèse ou comme intermédiaire de synthèse. C'est en particulier un réactif clé pour la préparation de la cyanhydrine d'acétone, intermédiaire de synthèse pour la production de méthacrylate de méthyle MAM, monomère de base des polymères thermoplastiques tels que le PMMA (Altuglas^{®}, Plexiglas^{®}). L'acide cyanhydrique est utilisé aussi dans la synthèse de la méthionine, ou pour fabriquer l'adiponitrile intermédiaire de synthèse du polyamide 6.6 (Nylon^{®}) et de nombreux agents chélatants. Le cyanure de sodium, dérivé de HCN possède également de nombreuses applications dans l'industrie chimique.

La production industrielle d'acide cyanhydrique HCN actuelle est basée principalement sur le procédé Andrussow datant des années 1930. Ce procédé consiste à faire réagir du méthane ou du gaz naturel sur de l'ammoniac en présence d'air et éventuellement d'oxygène sur un catalyseur composé de toiles de platine rhodié. La réaction CH₄ + NH₃ → HCN + 3 H₂ (1) étant endothermique, l'addition d'air permet, grâce à la combustion d'une partie de l'hydrogène produit et de l'excès de méthane, d'avoir un système globalement exothermique et ainsi d'entretenir la réaction de synthèse sans apport d'énergie extérieure.

La réaction, connue sous le nom d'ammoxydation, est la suivante :

CH₄ + NH₃ + 3/2 O₂ → HCN + 3 H₂O + chaleur (2)

Le procédé est basé sur les réactions (1) et (2).

Du fait de l'extrême sensibilité du catalyseur à un empoisonnement par certaines impuretés (fer, soufre...) la qualité des matières premières doit être la meilleure possible. On utilise notamment du méthane de pureté supérieure à 90% contenant le minimum d'hydrocarbures supérieurs (éthane et surtout propane) et exempt de soufre. L'ammoniac est filtré et évaporé et de préférence ne contient pas d'huiles ni de fer. L'air est dépoussiéré par un lavage à l'eau avant d'être comprimé.

Les trois réactifs (CH₄, NH₃, air) sont mélangés dans des proportions stoechiométriques précises. Le courant gazeux résultant est, après avoir été filtré, introduit dans le réacteur. Celui-ci est constitué de toiles de platine rhodié posées sur un support et d'une chaudière de trempe permettant de refroidir les gaz immédiatement après le contact avec le catalyseur. L'amorçage de la réaction est effectué grâce à un système de résistance électrique qui allume les toiles. Une fois cet allumage réalisé, l'exothermicité globale de la réaction maintient les toiles vers 1050 - 1150°C.

La cinétique est très rapide avec un temps de contact proche de quelques millisecondes ou dixièmes de millisecondes, et une vitesse des gaz de l'ordre de quelques mètres par seconde. La proportion de chaque réactif est optimisée de manière à obtenir un rendement maximal et éviter la zone d'inflammabilité du mélange réactionnel.

La réaction atteint généralement un rendement de 60 à 70 %, exprimé en nombre de moles d'acide cyanhydrique produites sur le nombre de moles d'ammoniac introduites, la conversion du méthane étant presque quantitative. La sélectivité en acide cyanhydrique est généralement de 80 à 90 % (nombre de moles d'HCN produites sur le nombre de moles de NH₃ ayant réagi).

Un autre procédé pour produire HCN, le procédé Degussa, est basé sur la réaction (1) précitée, en l'absence d'oxygène ou d'air, à une température de l'ordre de 1300°C. La réaction s'effectue alors dans des tubes d'alumine frittée revêtus intérieurement de platine, le faisceau de tubes étant chauffé au gaz à l'intérieur d'un four.

Dans ces procédés, une partie de l'ammoniac introduit pour réagir sur le méthane, ne participe pas à l'obtention de HCN, et, soit se décompose en azote et hydrogène selon la réaction :

2 NH₃ → N₂ + 3 H₂

soit reste inerte (by-pass).

La première étape de purification consiste alors à neutraliser l'ammoniac non converti par de l'acide sulfurique. La solution de sulfate d'ammonium ainsi générée est soumise à un stripage à la vapeur pour la débarrasser des traces d'HCN encore présentes.

HCN contenu dans les gaz débarrassés de NH₃ est ensuite absorbé dans de l'eau. En tête de cette colonne d'absorption ne restent plus que majoritairement des gaz inertes et de l'hydrogène qui sont dirigés vers un incinérateur. La solution aqueuse d'HCN est ensuite distillée. HCN sortant en tête est condensé à basse température. L'eau sortant en pied de cette colonne de purification, après avoir été refroidie, est recyclée vers l'absorbeur. Le produit obtenu à l'issue de ce procédé a une pureté supérieure à 99 % en masse.

Ces procédés, bien que conduisant à un produit de haute pureté présentent l'inconvénient d'être limité en productivité, car les rendements par rapport à l'ammoniac n'atteignent généralement que des valeurs de l'ordre de 60-70%.

Différentes solutions ont été recherchées pour augmenter le rendement de la réaction de l'ammoniac sur du méthane. Dans le brevet US 3,102,269 il est proposé d'ajouter une petite quantité d'un composé contenant du soufre volatil pendant les premières heures de la synthèse d'HCN pour réduire la période d'activation du catalyseur et augmenter la sélectivité. Le composé préféré est le disulfure de carbone (CS₂), mais d'autres composés contenant du soufre peuvent être utilisés, tels que le thiophène, les mercaptans, tels que méthyl, éthyl, propyl ou butyl mercaptan, les thioéthers tels que le diméthyl sulfure ou le diéthyl sulfure, ou le sulfure d'hydrogène. Le composé soufré est ajouté à une teneur équivalente à une teneur en soufre comprise entre 2 et 200 mg par m³ du mélange gazeux réactionnel, une teneur plus faible que 2 mg S/m³ ne donnant pas l'effet recherché d'activation, et une quantité plus importante, par exemple 500 mg, devenant un poison pour le catalyseur. Le rendement en HCN est passé de 61 % à 70 % avec l'ajout pendant 2 heures de l'équivalent de 5 mg de soufre/m³ gaz en CS₂.

Dans l'article Journal of catalysis, 22, (1971), pages 269-279, il est indiqué qu'après une production normale d'HCN pendant une période d'environ 1000 heures, 100 ppm de H₂S ont été ajoutés pendant une durée de 110 heures. Cela a eu pour effet d'augmenter le rendement en HCN de 4 % et en même temps la température des toiles de platine rhodié a augmenté de 20 °C.

Il est par ailleurs connu que le soufre pouvant être présent dans le méthane en quantité importante, par exemple sous forme de SO₂, H₂S, mercaptans ou de tétrahydrothiophène (agent odorant), est préjudiciable à la réaction d'ammoxydation. Le soufre est également connu pour modifier le diagramme de phase de l'alliage platine/rhodium en diminuant significativement son point de fusion et ainsi modifier les propriétés mécaniques du catalyseur en le fragilisant et limitant sa durée de vie (Massalski, Binary Alloy Diagrams, ASM International, Materials Park Ohio - 1991). Selon l'article « The Manufacture of Nitric Acid » extrait de la revue « Platinum Metals Rev., 1967, 11, (2), 60-69 » qui fait l'analogie avec le procédé HCN, il est fortement recommandé d'éviter la présence de composés soufrés dans la fabrication d'acide nitrique, fabrication qui met en oeuvre, tout comme le procédé HCN, une réaction entre de l'oxygène et de l'ammoniac sur une toile de platine rhodié. De tels composés soufrés peuvent par exemple se trouver dans les lubrifiants des compresseurs utilisés pour liquéfier l'ammoniac. Il est donc généralement recommandé de réduire la quantité de composés soufrés dans le lubrifiant à 5 ppm. De même l'ammoniac, s'il est de source non synthétique, peut contenir des quantités non négligeables de soufre, qui doivent être préalablement prélevées, pour que leur quantité n'excède pas 1 ou 2 ppm. De plus, l'air utilisé pour l'oxydation de l'ammoniac est généralement filtré pour lui soustraire les impuretés gazeuses de S0₂.

Le rôle du soufre dans la réaction de l'ammoniac avec du méthane apparaît complexe, bénéfique dans certaines conditions, mais aussi néfaste dans d'autres conditions. Les données dans la littérature sur la nature des composés soufrés ou leur quantité à utiliser sont par ailleurs contradictoires.

Il a maintenant été découvert de façon surprenante qu'un composé soufré appartenant à la famille des polysulfures, ajouté en faible quantité au cours de la production d'acide cyanhydrique selon le procédé Andrussow, ou le procédé Degussa augmente de façon significative le rendement en HCN par rapport à l'ammoniac. Cet effet est particulièrement marqué lorsque le composé soufré est un disulfure tel que le diméthyldisulfure. Un progrès même minime (c'est-à-dire de 1 à 5 %) du rendement du procédé de fabrication de HCN, a des conséquences extrêmement avantageuses en terme de gains de productivité. De plus l'activité des catalyseurs diminuant dans le temps en fonction des conditions d'utilisation, le rendement de fabrication de HCN a tendance à diminuer aussi avec le temps. On voit donc clairement l'intérêt d'une solution qui permet d'augmenter le rendement mais aussi d'augmenter la durée de vie du catalyseur pour améliorer la rentabilité de l'unité de production.

L'effet du diméthyldisulfure sur le rendement de la réaction s'est avéré nettement plus important que celui obtenu avec H₂S habituellement utilisé dans les unités industrielles.

Le diméthyldisulfure de formule H₃C-S-S-CH₃, ci-après dénommé DMDS, ou pouvant être appelé aussi disulfure de diméthyle ou méthyl dithiométhane, est employé dans un grand nombre d'applications. Notamment le DMDS est utilisé comme agent de sulfuration ou de pré-sulfuration dans les raffineries afin d'activer les catalyseurs d'hydrotraitement. Le DMDS est utilisé aussi dans l'industrie des produits pétrochimiques pour protéger les circuits de vapocraquage de la formation de coke et de monoxyde de carbone. Il peut être utilisé également comme intermédiaire de synthèse en chimie fine ou en métallurgie pour ses propriétés anti-corrosion.

Jusqu'à maintenant, les disulfures tel que le DMDS, ou plus généralement les polysulfures, n'ont jamais été utilisés dans un procédé de production d'acide cyanhydrique et son effet est tout à fait inattendu. Sans que la Demanderesse soit tenue à une quelconque explication, elle pense que dans les conditions opératoires du procédé Andrussow ou du procédé Degussa, le DMDS se décompose en différentes espèces chimiques qui se retrouvent en équilibre du fait de leurs faibles temps de séjour dans l'installation, améliorant l'efficacité du catalyseur, le rendement en acide cyanhydrique et réduisant la perte d'ammoniac par décomposition.

La présente invention a donc pour but de fournir un procédé de fabrication d'acide cyanhydrique, de rendement amélioré, qui permet de réduire la perte d'ammoniac par décomposition et par conséquent qui conduit à une capacité de production plus importante et/ou des coûts de production moindres.

Le but de la présente invention est aussi de permettre une durée d'activation du catalyseur plus courte, et une durée d'utilisation du catalyseur plus longue en gardant le même rendement élevé plus longtemps, ce qui permet d'améliorer la rentabilité de l'unité de production.

La présente invention a également pour but de fournir un procédé amélioré de fabrication d'acide cyanhydrique qui soit simple, rapide (comportant le moins d'étapes possibles), facile à mettre en oeuvre, et qui s'adapte aisément aux dispositifs de fabrication d'acide cyanhydrique existants dans l'industrie.

La présente invention a pour objet un procédé de production d'acide cyanhydrique dans lequel on fait passer un mélange gazeux comprenant du méthane (ou un gaz naturel) et de l'ammoniac, et éventuellement de l'air et/ou de l'oxygène sur un catalyseur au platine, caractérisé en ce que l'on ajoute dans le mélange gazeux au moins un composé soufré répondant à la formule générale (l) : R - S - (S)ₓ - R' dans laquelle R et R', identiques ou différents, représentent un radical alkyle ou alcényle, linéaire ou ramifié, contenant de 1 à 5 atomes de carbone, et x est un nombre allant de 1 à 5.

Selon un mode de réalisation préféré de l'invention, on fait passer un mélange gazeux comprenant du méthane (ou un gaz naturel), de l'ammoniac, de l'air et éventuellement de l'oxygène sur un catalyseur composé de toile de platine rhodié.

Selon un autre mode de réalisation préféré de l'invention, on fait passer un mélange gazeux comprenant du méthane (ou un gaz naturel) et de l'ammoniac dans des tubes d'alumine frittée revêtus intérieurement de platine à une température de l'ordre de 1300°C.

Comme exemples non limitatifs de radicaux R et R', on peut citer les radicaux méthyle, éthyle, propyle, allyle et propényle. De préférence, les radicaux R ou R' sont les radicaux méthyle, éthyle ou propyle. Parmi les composés de formule (I), on préfère ceux pour lesquels x va de 1 à 3, de préférence les disulfures (x=1), et plus particulièrement le diméthyldisulfure (DMDS).

Le diméthyldisulfure (DMDS) est un produit largement disponible, il est notamment commercialisé par la société Arkema.

Le procédé selon l'invention se caractérise en ce qu'il comprend l'ajout d'une certaine quantité de composé soufré répondant à la formule (I). Le composé soufré peut-être ajouté directement à l'une au moins des matières brutes, méthane ou gaz naturel, ammoniac, ou air ou oxygène, en amont du mélange. Le composé soufré peut aussi être ajouté directement dans le mélange gazeux, méthane / ammoniac, ou méthane / ammoniac / air et/ou oxygène, au niveau du mélangeur ou en aval du mélangeur dans le courant gazeux avant son passage sur le catalyseur. Selon le procédé de l'invention, il est possible d'utiliser une seule de ces possibilités d'ajout ou bien de combiner plusieurs de ces différentes possibilités, le composé soufré pouvant être ajouté par injection en un ou plusieurs points d'injection du procédé.

L'ajout de composé soufré se fait de préférence pendant la marche normale de la réaction bien qu'il soit possible également de l'ajouter pendant l'étape d'activation du catalyseur (24 à 48 heures environ).

Le composé soufré de formule (I) est de préférence ajouté en continu afin de maintenir un niveau optimal de soufre. Le composé soufré peut être ajouté en continu sur une durée supérieure à 30 jours de marche de l'installation.

Les quantités de composé soufré de formule (I) injectées dans le mélange gazeux vont de 5 à 500 ppm exprimés en volume de soufre par rapport au volume de méthane introduit, de préférence de 5 à 200 ppm de soufre et plus particulièrement de 5 à 100 ppm, et encore plus préférentiellement de 5 à 50 ppm exprimés en volume de soufre par rapport au volume de méthane. Ces quantités de composé soufré n'ont pas d'impact néfaste pour l'utilisation ultérieure du produit obtenu à partir du procédé selon l'invention.

Tous les autres paramètres opératoires du procédé peuvent être maintenus constants, par rapport au procédé sans ajout de composé soufré de formule (I). Typiquement, dans un procédé Andrussow, on utilise du méthane de pureté 95 % environ, le rapport molaire CH₄ / NH₃ va de 1,0 à 1,2, le rapport molaire (CH₄+NH₃) / O₂ total va de 1,5 à 2, de préférence de 1,6 à 1,9 ; la pression est généralement de 1 à 2 bar ; la température de réaction est comprise entre 1050°C et 1150°C.

Dans ces conditions de paramètres maintenus constants (puretés des matières premières, rapports molaires constants, température et pression constantes, temps de séjour constant....), l'effet du composé soufré de formule (I) tel que le DMDS se traduit par une augmentation du rendement en HCN de 1 à 5 % par rapport à l'ammoniac introduit, une augmentation de la sélectivité liée à une diminution du taux de décomposition de l'ammoniac et une augmentation de la température du catalyseur de 10 à 40°C. Avantageusement, la quantité d'oxygène introduite le cas échéant peut alors être diminuée, ainsi que les quantités de méthane et d'ammoniac, ce qui a pour conséquence une augmentation de la productivité.

Le procédé de l'invention permet de s'affranchir de l'utilisation d'un gaz toxique tel que H₂S et fait appel à un produit liquide non toxique facilement vaporisable dans les conditions du procédé (point d'ébullition d'environ 110°C). Le composé soufré de formule (I), tel que le DMDS permet d'améliorer significativement la productivité du catalyseur mis en oeuvre et ce, sans nécessiter d'étape supplémentaire de purification du produit fini. Contrairement à l'utilisation du H₂S, le produit obtenu selon le procédé de l'invention est exempt de composé soufré tel que H₂S, ce qui permet son utilisation directe dans tout procédé ultérieur dans lequel la présence de soufre n'est pas souhaitable, tel qu'un procédé de préparation de cyanhydrine d'acétone.

De façon surprenante, il a été constaté par ailleurs que le composé soufré de formule (I) tel que le DMDS a moins d'effets négatifs à long terme sur le catalyseur, notamment d'un point de vue fragilité des toiles de platine rhodié ou perte de métal, que d'autres composés soufrés tels que H₂S ou diméthylsulfure (DMS). Les catalyseurs peuvent donc être utilisés sur une durée nettement plus longue avant d'être changés.

L'invention porte aussi sur l'utilisation d'au moins un composé soufré répondant à la formule générale (I) : R - S - (S)ₓ, - R' dans laquelle R et R', identiques ou différents, représentent un radical alkyle ou alcényle, linéaire ou ramifié, contenant de 1 à 5 atomes de carbone, et x est un nombre allant de 1 à 5, en quantité efficace dans un procédé de production d'acide cyanhydrique par réaction d'ammoniac et de méthane (ou de gaz naturel), pour augmenter le rendement dudit procédé.

Le produit obtenu directement à partir du procédé selon l'invention est avantageusement utilisé pour produire de la méthionine ou l'hydroxyanalogue de la méthionine par réaction avec du méthylmercaptopropionaldéhyde (MMP).

La méthionine, ou acide 2-amino-4-(méthylthio) butyrique, de formule chimique CH₃-S-(CH₂)₂-CH(NH₂)-COOH est un acide aminé essentiel, non synthétisé par les animaux, nécessaire comme appoint dans la ration alimentaire, notamment de la volaille, dont les besoins en méthionine sont importants. La méthionine obtenue par voie de synthèse chimique s'est imposée comme substitut des apports d'origine naturelle (farines de poisson, tourteaux de soja...) pour l'alimentation animale, principalement pour la volaille.

Contrairement aux autres acides aminés, la méthionine est assimilable biologiquement aussi bien sous la forme dextrogyre (d ou +) que sous la forme lévogyre (I ou -), ce qui a permis le développement de synthèses chimiques conduisant au produit racémique. Ainsi le marché de la méthionine de synthèse est principalement celui de la dl-méthionine, produit solide couramment désigné par DLM. Il existe également un dérivé liquide de la méthionine, l'α-hydroxy acide, correspondant à l'acide 2-hydroxy-4-(méthylthio) butyrique de formule chimique CH₃-S-(CH₂)₂-CH(OH)-COOH, qui a la particularité d'être transformé in vivo en méthionine de façon pratiquement quantitative. Ce produit liquide, disponible commercialement sous forme de solution aqueuse à 88% en masse, est désigné couramment par hydroxyanalogue de la méthionine.

De nombreuses synthèses ont été décrites concernant la méthionine ou son dérivé hydroxylé, mais les procédés chimiques exploités industriellement reposent essentiellement sur les mêmes matières premières principales et les mêmes intermédiaires clés, à savoir :
- l'acroléine et le méthyl mercaptan (MSH) conduisant au méthylmercaptopropionaldéhyde (MMP), désigné aussi par 3-(methylthio)propanal ou par aldéhyde méthylthiopropionique (AMTP),
- l'acide cyanhydrique ou le cyanure de sodium (NaCN), qui après réaction avec le MMP, conduit finalement à la méthionine ou à l'hydroxyanalogue de la méthionine.

On pourra se reporter à l'article Techniques de l'Ingénieur, traité Génie des Procédés, J 6-410-1 à 9 qui décrit les conditions de mise en oeuvre industrielle des procédés de synthèse de la méthionine utilisant le méthylmercaptopropionaldéhyde et l'acide cyanhydrique comme produits intermédiaires, un des procédés pouvant être illustré schématiquement par les réactions suivantes :

H₂S + CH₃OH → CH₃SH + H₂O

CH₃SH + CH₂=CH-CHO → CH₃-S-CH₂-CH₂-CHO

CH₃-S-CH₂-CH₂-CHO + HCN → CH₃-S-CH₂-CH₂-CH(OH)-CN

CH₃-S-CH₂-CH₂-CH(OH)-CN + NH₃ → CH₃-S-CH₂-CH₂-CH(NH₂)-CN

CH₃-S-CH₂-CH₂-CH(NH₂)-CN +2 H₂O +H⁺ → CH₃-S-CH₂)₂-CH(NH₂)-COOH.

Avantageusement, le produit obtenu directement à partir du procédé selon l'invention est utilisé aussi pour produire de la cyanhydrine d'acétone par réaction avec de l'acétone selon la réaction :

CH₃-C(O)-CH₃ + HCN → (CH₃)₂C(OH)CN

L'acétone cyanhydrine est un composé intermédiaire pour produire du méthacrylate de méthyle (MAM) selon les deux voies schématisées ci-après. Une première voie consiste à former de l'α-oxyisobutyramide monosulfate, qui se transforme en méthacrylamide sulfurique. Ce dernier est ensuite hydrolysé et estérifié par le méthanol pour former le méthacrylate de méthyle.

Une seconde voie consiste à faire réagir directement du méthanol, puis à mettre en oeuvre une réaction de déshydratation pour conduire au méthacrylate de méthyle.

On pourra se reporter à l'article Techniques de l'Ingénieur, traité Génie des Procédés, J 6-400-1 à 6 qui décrit les conditions de mise en oeuvre industrielle du procédé de production de méthacrylate de méthyle selon la voie cyanhydrine d'acétone.

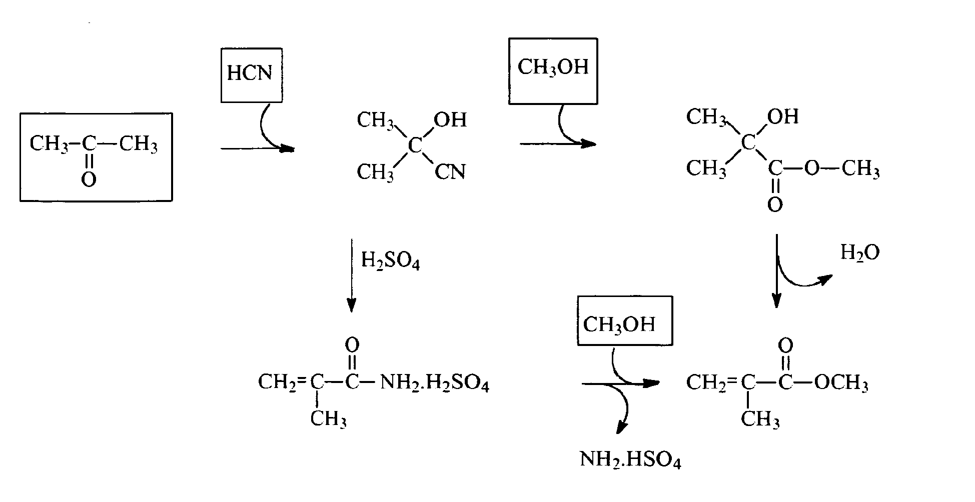

Avantageusement, le produit obtenu directement à partir du procédé selon l'invention est utilisé aussi pour produire de l'adiponitrile par réaction avec du butadiène selon la réaction :

CH₂=CH-CH=CH₂ + 2 HCN → NC-(CH₂)₄-CN

L'adiponitrile, après hydrogénation conduit à l'hexaméthylènediamine qui est un composé intermédiaire pour produire le polyamide 6-6 (Nylon^{®}) par polycondensation de l'adipate d'hexaméthylènediamine.
On pourra se reporter à l'article Techniques de l'Ingénieur, traité Génie des Procédés, J 6-515-1 à 7 qui décrit la synthèse de polyamide 6-6 selon cette voie.

Avantageusement, le produit obtenu directement à partir du procédé selon l'invention est utilisé aussi pour produire du cyanure de sodium par neutralisation avec de l'hydroxyde de sodium selon la réaction :

HCN + Na OH → Na CN + H₂O

Le cyanure de sodium possède de nombreuses applications, notamment pour l'extraction de métaux précieux, la galvanoplastie ou la synthèse de composés chimiques.

Les exemples qui suivent illustrent la présente invention sans toutefois en limiter la portée.

### EXEMPLES

### Exemple 1

Du Gaz Naturel (GN), sans composé soufré et titrant 95 % volume en méthane, est mélangé à de l'ammoniac, de l'air et de l'oxygène dans des proportions volumiques CH₄/NH₃ de 1,16 et (CH₄ +NH₃)/O₂ total de 1,70. Le débit en GN est de 4000 kg/h. Le mélange est envoyé à travers un lit de 15 toiles Pt/Rh (90/10). Le rendement en HCN par rapport à l'ammoniac se stabilise à 68,0 % après 48 heures, la température est alors d'environ 1060°C.

Du DMDS est ajouté à raison de 10 ppm exprimés en volume de soufre par rapport au volume de méthane. Très rapidement, le rendement augmente à 70,0 %, le taux de décomposition de l'ammoniac, déterminé à partir de l'analyse des gaz N₂ et H₂ en sortie, chute de 2 %, et la température des toiles augmente de + 10°C. Le DMDS est injecté en continu ce qui permet de maintenir les performances pendant plus de 60 jours. L'arrêt de l'injection du DMDS provoque une chute progressive du rendement. L'HCN pur produit ne contient pas de composé soufré.

### Exemple 2

Le même protocole que l'exemple 1 est reproduit sauf que le DMDS est ajouté à raison de 125 ppm exprimés en volume de soufre par rapport au volume de méthane. Très rapidement, le rendement augmente à 73,0 %, le taux de décomposition de l'ammoniac chute de 4 % et la température des toiles augmente de + 40°C. Le DMDS est injecté en continu ce qui permet de maintenir les performances pendant plus de 50 jours. L'HCN pur produit ne contient pas de composé soufré.

### Exemple 3 (comparatif)

Le même protocole que l'exemple 1 est reproduit sauf que l'on ajoute de l'H₂S à la place du DMDS. L'H₂S est ajouté à raison de 100 ppm exprimés en volume de soufre par rapport au volume de méthane Le rendement en HCN n'augmente que de 1,0 %, le taux de décomposition de l'ammoniac et la température des toiles restent pratiquement inchangés. De plus, l'HCN pur produit contient des traces d'H₂S néfastes pour l'application en aval.

### Exemple 4

Du Gaz Naturel (GN), sans composé soufré et titrant 95 % volume en méthane, est mélangé à de l'ammoniac, de l'air et de l'oxygène dans des proportions volumiques CH₄/NH₃ de 1,16 et (CH₄ +NH₃)/O₂ total de 1,73. Le débit en GN est de 4100 kg/h. Le mélange est envoyé à travers un lit de 18 toiles Pt/Rh (90/10). Le rendement en HCN par rapport à l'ammoniac est de 67 % après 50 jours de run, la température est alors d'environ 1060°C.

Du DMDS est ajouté à raison de 15 ppm exprimés en volume de soufre par rapport au volume de méthane. Très rapidement, le rendement augmente à 69 %, le taux de décomposition de l'ammoniac, chute de 3,5 %, et la température des toiles augmente de + 10°C.

L'arrêt de l'injection du DMDS provoque une chute du rendement à 67 %, une augmentation du taux de décomposition de l'ammoniac de 3,5% et une chute de la température de 10°C.

Le DMDS est à nouveau injecté à raison de 25 ppm exprimés en volume de soufre par rapport au volume de méthane. Très rapidement, le rendement augmente à 69 %, le taux de décomposition de l'ammoniac chute de 3,5 %, et la température des toiles augmente de 10°C.

### Exemple 5

Dans un autre réacteur que celui des exemples précédents, du Gaz Naturel (GN), sans composé soufré et titrant 97 % volume en méthane, est mélangé à de l'ammoniac, de l'air et de l'oxygène dans des proportions volumiques CH₄/NH₃ de 1,09 et (CH₄ +NH₃)/O₂ total de 1,95. Le débit en GN est de 3120 kg/h. Le mélange est envoyé à travers un lit de 20 toiles Pt/Rh (90/10). Le rendement en HCN par rapport à l'ammoniac se stabilise à 64,0 % après une semaine de fonctionnement.

Du DMDS est ajouté à raison de 10 ppm exprimés en volume de soufre par rapport au volume de méthane. Très rapidement, le rendement augmente à 67,0 %, le taux de décomposition de l'ammoniac chute, la température des toiles augmente. Le DMDS est injecté en continu ce qui permet, malgré des variations de débit d'ammoniac et de gaz naturel, de maintenir le rendement à au moins 67 % pendant 60 jours. Un rendement de 68 % est même obtenu en augmentant la concentration en DMDS à 20 ppm exprimés en volume de soufre par rapport au volume de méthane. L'arrêt de l'injection du DMDS provoque une chute du rendement.

### Exemple 6

Du Gaz Naturel (GN), sans composé soufré et titrant 94 % volume en méthane, dopé avec du DMDS à raison de 20 ppm exprimés en volume de soufre par rapport au volume de méthane, est mélangé à de l'ammoniac, de l'air et de l'oxygène dans des proportions volumiques CH₄/NH₃ de 1,15 et (CH₄ +NH₃)/O₂ total de 1,76. Le débit en GN est de 3840 kg/h. Le mélange est envoyé à travers un lit de 20 toiles Pt/Rh (90/10). Le rendement en HCN par rapport à l'ammoniac est de 66,5 %, la température est alors d'environ 1060°C. L'arrêt de l'injection du DMDS provoque une chute du rendement à 64 %, une augmentation du taux de décomposition de l'ammoniac de 3,5 % et une chute de la température de 10°C.

L'H₂S est ensuite ajouté à raison de 3 ppm exprimés en volume de soufre par rapport au volume de méthane. Le rendement en HCN augmente de moins de 1,0 %, le taux de décomposition de l'ammoniac et la température des toiles restent pratiquement inchangés.

L'arrêt de l'injection d'H₂S provoque une faible chute du rendement à 64 %.

## Revendications

1. Procédé de production d'acide cyanhydrique dans lequel on fait passer un mélange gazeux comprenant du méthane (ou un gaz naturel) et de l'ammoniac, et éventuellement de l'air et/ou de l'oxygène, sur un catalyseur au platine, **caractérisé en ce que** l'on ajoute dans le mélange gazeux au moins un composé soufré répondant à la formule générale (I) R - S - (S)ₓ - R' dans laquelle R et R', identiques ou différents, représentent un radical alkyle ou alcényle, linéaire ou ramifié, contenant de 1 à 5 atomes de carbone, et x est un nombre allant de 1 à 5, de préférence de 1 à 3.

2. Procédé selon la revendication 1 **caractérisé en ce que** les radicaux R et R' sont choisis parmi les radicaux méthyle, éthyle ou propyle.

3. Procédé selon la revendication 1 ou 2 **caractérisé en ce que** le composé soufré est un disulfure, de préférence le diméthyldisulfure.

4. Procédé selon l'une quelconque des revendications précédentes **caractérisé en ce que** le composé soufré est ajouté directement à l'une au moins des matières brutes, méthane ou gaz naturel, ammoniac, ou air ou oxygène, en amont du mélange.

5. Procédé selon l'une quelconque des revendications 1 à 3 **caractérisé en ce que** le composé soufré est ajouté directement dans le mélange gazeux au niveau du mélangeur.

6. Procédé selon l'une quelconque des revendications 1 à 3 **caractérisé en ce que** le composé soufré est ajouté dans le mélange gazeux en aval du mélangeur dans le courant gazeux avant son passage sur le catalyseur.

7. Procédé selon l'une quelconque des revendications précédentes **caractérisé en ce que** la quantité de composé soufré ajoutée va de 5 à 500 ppm exprimée en volume de soufre par rapport au volume de méthane, de préférence de 5 à 100 ppm en volume de soufre par rapport au volume de méthane.

8. Procédé selon l'une quelconque des revendications précédentes **caractérisé en ce que** le composé soufré est injecté en continu.

9. Utilisation d'au moins un composé soufré répondant à la formule générale (I) R - S - (S)ₓ - R' dans laquelle R et R', identiques ou différents, représentent un radical alkyle ou alcényle, linéaire ou ramifié, contenant de 1 à 5 atomes de carbone, et x est un nombre allant de 1 à 5, en quantité efficace dans un procédé de production d'acide cyanhydrique par réaction d'ammoniac et de méthane (ou de gaz naturel), pour augmenter le rendement dudit procédé.

## Claims

1. Process for producing hydrocyanic acid in which a gas mixture comprising methane (or a natural gas) and ammonia and optionally air and/or oxygen, is passed over a platinum catalyst, **characterized in that** added to the gas mixture is at least one sulphur-containing compound corresponding to the general formula (I): R-S-(S)ₓ-R' in which R and R', which are identical or different, represent a linear or branched alkyl or alkenyl radical containing from 1 to 5 carbon atoms and x is a number ranging from 1 to 5, preferably from 1 to 3.

2. Process according to Claim 1, **characterized in that** the radicals R and R' are chosen from methyl, ethyl or propyl radicals.

3. Process according to Claim 1 or 2, **characterized in that** the sulphur-containing compound is a disulphide, preferably dimethyl disulphide.

4. Process according to any one of the preceding claims, **characterized in that** the sulphur-containing compound is added directly to at least one of the raw materials, methane or natural gas, ammonia, or air or oxygen, upstream of the mixing.

5. Process according to any one of Claims 1 to 3, **characterized in that** the sulphur-containing compound is added directly to the gas mixture at the mixer.

6. Process according to any one of Claims 1 to 3, **characterized in that** the sulphur-containing compound is added to the gas mixture downstream of the mixer into the gas stream before it passes over the catalyst.

7. Process according to any one of the preceding claims, **characterized in that** the amount of sulphur-containing compound added ranges from 5 to 500 ppm expressed by volume of sulphur relative to the volume of methane, preferably from 5 to 100 ppm by volume of sulphur relative to the volume of methane.

8. Process according to any one of the preceding claims, **characterized in that** the sulphur-containing compound is injected continuously.

9. Use of at least one sulphur-containing compound corresponding to the general formula (I): R-S-(S)ₓ-R' in which R and R', which are identical or different, represent a linear or branched alkyl or alkenyl radical containing from 1 to 5 carbon atoms and x is a number ranging from 1 to 5, in an effective amount in a process for producing hydrocyanic acid by reaction of ammonia and methane (or natural gas), in order to increase the yield of said process.

## Patentansprüche

1. Verfahren zur Herstellung von Cyanwasserstoffsäure, bei dem man eine Methan (oder Erdgas) und Ammoniak und gegebenenfalls Luft und/oder Sauerstoff umfassende Gasmischung über einen Platinkatalysator leitet, **dadurch gekennzeichnet, daß** man der Gasmischung mindestens eine Schwefelverbindung der allgemeinen Formel (I) R - S - (S)ₓ - R', worin R und R' gleich oder verschieden sind und für einen linearen oder verzweigten Alkyl- oder Alkenylrest mit 1 bis 5 Kohlenstoffatomen stehen und x für eine Zahl von 1 bis 5, vorzugsweise von 1 bis 3, steht, zusetzt.

2. Verfahren nach Anspruch 1, **dadurch gekennzeichnet, daß** die Reste R und R' unter Methyl-, Ethyl- oder Propylresten ausgewählt sind.

3. Verfahren nach Anspruch 1 oder 2, **dadurch gekennzeichnet, daß** es sich bei der Schwefelverbindung um ein Disulfid, vorzugsweise Dimethyldisulfid, handelt.

4. Verfahren nach einem der vorhergehenden Ansprüche, **dadurch gekennzeichnet, daß** man die Schwefelverbindung vor dem Mischen direkt mindestens einem der Ausgangsstoffe, Methan oder Erdgas, Ammoniak oder Luft oder Sauerstoff, zusetzt.

5. Verfahren nach einem der Ansprüche 1 bis 3, **dadurch gekennzeichnet, daß** man die Schwefelverbindung auf Höhe des Mischers direkt zu der Gasmischung gibt.

6. Verfahren nach einem der Ansprüche 1 bis 3, **dadurch gekennzeichnet, daß** die Zugabe der Schwefelverbindung zu der Gasmischung hinter dem Mischer in den Gasstrom vor dessen Übergang über den Katalysator erfolgt.

7. Verfahren nach einem der vorhergehenden Ansprüche, **dadurch gekennzeichnet, daß** die zugesetzte Menge der Schwefelverbindung 5 bis 500 ppm, ausgedrückt als Schwefelvolumen in Bezug auf das Methanvolumen, vorzugsweise 5 bis 100 Vol.-ppm Schwefel, bezogen auf das Methanvolumen, beträgt.

8. Verfahren nach einem der vorhergehenden Ansprüche, **dadurch gekennzeichnet, daß** man die Schwefelverbindung kontinuierlich einleitet.

9. Verwendung mindestens einer Schwefelverbindung der allgemeinen Formel (I) R-S-(S)ₓ-R', worin R und R' gleich oder verschieden sind und für einen linearen oder verzweigten Alkyl- oder Alkenylrest mit 1 bis 5 Kohlenstoffatomen stehen und x für eine Zahl von 1 bis 5 steht, in einer wirksamen Menge bei einem Verfahren zur Herstellung von Cyanwasserstoffsäure durch Umsetzung von Ammoniak und Methan (oder Erdgas) zur Erhöhung der Ausbeute des Verfahrens.
